(19) Europäisches Patentamt
European Patent Office
Office européen des brevets

(11) Veröffentlichungsnummer: **0 164 046**
**B1**

(12) **EUROPÄISCHE PATENTSCHRIFT**

(45) Veröffentlichungstag der Patentschrift: **13.09.89**

(51) Int. Cl.⁴: **C 07 C 85/24**, C 07 C 87/56

(21) Anmeldenummer: **85106496.4**

(22) Anmeldetag: **25.05.85**

(54) **Verfahren zur Isomerisierung von o-, m-, und/oder p-Toluidin.**

(30) Priorität: **02.06.84 DE 3420707**

(43) Veröffentlichungstag der Anmeldung:
**11.12.85 Patentblatt 85/50**

(45) Bekanntmachung des Hinweises auf die Patenterteilung:
**13.09.89 Patentblatt 89/37**

(84) Benannte Vertragsstaaten:
**BE CH DE FR GB IT LI NL**

(56) Entgegenhaltungen:
**EP-A-0 072 008**
**EP-A-0 077 523**
**EP-A-0 092 103**

(73) Patentinhaber: **HOECHST AKTIENGESELLSCHAFT, Postfach 80 03 20, D-6230 Frankfurt am Main 80 (DE)**

(72) Erfinder: **Eichler, Klaus, Dr., Im Traminer 10, D-6236 Eschborn 2 (DE)**
Erfinder: **Leupold, Ernst Ingo, Dr., Am Zäunefeld 15, D-6392 Neu- Anspach (DE)**
Erfinder: **Arpe, Hans- Jürgen, Prof. Dr., de- Ridder-Weg 10, D-6230 Frankfurt am Main 80 (DE)**
Erfinder: **Baltes, Herbert, Dr., Johannesallee 24, D-6230 Frankfurt am Main 80 (DE)**

**EP 0 164 046 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Isomerisierung von o-, m-, und/oder p-Toluidin an einem Zeolith-Katalysator.

o-, m- und p-Toluidin (Methylaniline, Aminotoluole, Toluolamine), d.h. $C_6H_4CH_3NH_2$-Isomere werden technisch zumeist durch Reduktion der entsprechenden Nitrotoluole hergestellt. o-, m- und p-Nitrotoluol wiederum entstehen nebeneinander bei der Nitrierung von Toluol. So erhält man beispielsweise unter üblichen Nitrierbedingungen ein Gemisch von 63 % o-, 33 - 34 % p- und 3 - 4 % m-Nitrotoluol, das durch eine Kombination von Destillation und Kristallisation mit einer speziellen Methodik, des sog. Abtropfens, in die Reinprodukte zerlegt werden kann (Winnacker-Küchler, Carl Hanser Verlag München 1972, Band 4, S. 156). Das Verhältnis der Isomeren ist durch verschiedene Maßnahmen beeinflußbar (Kirk-Othmer, John Wiley, 3. Aufl., Band 15, S. 929); so kann beispielsweise der Gewichtsanteil des p-Nitrotoluols zwischen ca. 63 - 38 % und der des o-Nitrotoluols zwischen 34 - 58 % schwanken. Dagegen kann der Anteil des m-Nitrotoluols nur in sehr begrenztem Maß, nämlich zwischen 2,3 und 4,3 Gew.-% der Gesamt-Nitrotoluol-Ausbeute variiert werden. Damit steht besonders das m-Nitrotoluol für die weitere Reduktion zu m-Toluidin nur in begrenztem Maß zur Verfügung.

Es ist bereits bekannt, daß sich o-, m- und p-Toluidin oder ein Gemisch aus diesen an Zeolith-Katalysatoren isomerisieren läßt, wobei Zeolithe vom Pentasil-Typ bevorzugt werden (EP-A1-92 103). Allerdings zeigt der dort beschriebene Katalysator mit der Zeit eine deutliche Aktivitätsabnahme, die wahrscheinlich auf eine Verkokung zurückzuführen ist. Eine derartige Verkokung ist von Nachteil für die Durchführung eines technischen Prozesses, da der Katalysator oft regeneriert werden muß.

Es wurde nun gefunden, daß bei der Isomerisierung von Toluidinen an zirkonhaltigen Zeolithen vom Pentasil-Typ, wie sie z. B. in EP-A2-77 523 beschrieben werden, diese Aktivitätsabnahme deutlich geringer ist und nach einer anfänglichen kurzen Abnahme der Aktivität über einen langen Zeitraum sogar völlig konstante und höhere Ausbeuten erhalten werden können als mit den bisher beschriebenen Katalysatoren.

Gegenstand der Erfindung ist daher ein Verfahren zur Isomerisierung von o-, m- oder p-Toluidin oder einem Gemisch derselben an einem Zeolith-Katalysator, dadurch gekennzeichnet, daß ein zirkonhaltiger Zeolith vom Pentasil-Typ bei Temperaturen zwischen 300 und 550°C und Drücken zwischen 0,1 und 30 bar verwendet wird. Insbesondere ist Gegenstand der vorliegenden Erfindung ein Verfahren zur Herstellung von m-Toluidin durch Isomerisierung von o- oder p-Toluidin oder einem Gemisch derselben an einem solchen Zeolith.

Aufgrund des Standes der Technik war es überraschend und in keiner Weise vorhersehbar, daß sich an zirkonhaltigen Zeolithen des Pentasil-Typs höhere Isomerisierungsausbeuten bei geringerer Desaktivierung des Katalysators erzielen lassen als mit den vorbeschriebenen zirkonfreien Zeolithen. Der Vergleich zwischen Beispiel 1 und dem Vergleichsbeispiel zeigt, daß mit den erfindungsgemäßen Katalysatoren unter gleichen Bedingungen erheblich höhere Isomerisierungsausbeuten erhalten werden. Darüber hinaus wird in Beispiel 1 über 120 Stunden keine Änderung der katalytischen Aktivität beobachtet, während mit den bisher bekannten Katalysatoren in diesem Zeitraum ein deutliches Absinken der Aktivität durch Katalysatordesaktivierung stattfindet, wie auch das Vergleichsbeispiel zeigt.

Zur Durchführung des erfindungsgemäßen Verfahrens wird o-, m- oder p-Toluidin oder ein Gemisch aus zweien oder allen dreien dieser Isomeren mit einem zirkonhaltigen Zeolith-Katalysator vom Pentasil-Typ in Kontakt gebracht. Setzt man ein Gemisch ein, das alle drei Isomeren enthält, so kann natürlich nur dann eine Isomerisierung beobachtet werden, wenn die Zusammmensetzung des Ausgangsgemisches bei der Reaktionstemperatur abweicht von der eines Toluidin-Gemisches, daß sich im thermodynamischen Gleichgewicht befindet. Als Katalysatoren sind Zirkonosilikate und Zirkonoaluminosilikate mit Pentasil-Struktur geeignet.

Für den Begriff Pentasile gilt dabei die Definition von Kokotailo und Meier ("Pentasil family of high silicon crystalline materials" in Special Publication No. 33 of the Chemical Society, London, 1980). Die Pentasil-Familie umfaßt beispielsweise die synthetischen Zeolithe ZSM-5 (US-PS-3 702 886), ZSM-8 (GB-PS-1 334 243), ZSM-11 (US-PS-3 709 979) und Z5M-23 (US-PS-4 076 842).

Beim erfindungsgemäßen Verfahren sind vor allem Zirkonosilicate und Zirkonoaluminosilicate mit Z5M-5-Struktur geeignet, vorzugsweise solche mit folgender Zusammensetzung, ausgedrückt in Molverhältnissen der Oxide:

$SiO_2$ : (0 - 0,15) $Al_2O_3$ : (0,002 - 1,0) $ZrO_2$, insbesondere
$SiO_2$ : (0 - 0,1) $Al_2O_3$ : (0,01 - 0,4) $ZrO_2$ (siehe EP-A2-77 523).

Diese zirkonhaltigen Zeolithe lassen sich nach den gleichen Methoden und unter Einsatz der gleichen organischen Verbindungen herstellen, wie sie auch für die Synthese des zirkonfreien Zeolithen ZSM-5 beschrieben wurden, beispielsweise unter Verwendung von

Alkylammoniumverbindungen (US-PS-3 702 886)
Alkylaminen (US-PS-4 151 189)
Alkyldiaminen (DE-OS-2 817 576, DE-OS-2 831 334)
Alkylaminen in Gegenwart von Alkylierungsmitteln (EP-OS-11 362, DE-AS-2 212 810)
Aminoalkoholen (GB-PS-2 023 562)
Alkoholen (DE-OS-2 935 123, US-PS-4 175 114, EP-OS-42 225, DE-OS-2 643 929)

Ethern (EP-OS-51 741)

Vorzugsweise verwendet man Alkylammoniumverbindungen, Alkyldiamine, oder Alkylamine in Gegenwart von Alkylierungsmitteln. Unter den Alkylammoniumverbindungen sind besonders bevorzugt die Tetrapropylammoniumverbindungen, beispielsweise das Hydroxid oder eines der Halogenide. Ein besonders geeignetes Alkyldiamin ist Hexamethylendiamin.

Zur Synthese der zirkonhaltigen Pentasile mischt man eine oder mehrere Verbindungen aus den genannten Klassen mit Zirkon-Verbindungen und mit Silizium- und Natriumverbindungen und Wasser - sowie im Falle der Aluminosilicate noch zusätzlich mit Aluminiumverbindungen - und erhitzt dieses Gemisch in einem geschlossenen Gefäß. Dem Gemisch werden vorzugsweise darüberhinaus vor dem Erhitzen Impfkristalle eines Pentasils zugesetzt.

Falls man Tetrapropylammoniumverbindungen verwendet, werden die Ausgangsverbindungen im allgemeinen in folgendem Verhältnis eingesetzt, ausgedrückt in Molverhältnissen der Oxide:

$SiO_2$ : (0 - 0,2) $Al_2O_3$ : (0,01 - 1,0) $ZrO_2$ : (0,01 - 0,5) $Na_2O$ : (0,02 - 1,0) $R_2O$: (5 - 100) $H_2O$,

vorzugsweise im Verhältnis

$SiO_2$ : (0 - 0,1) $Al_2O_3$ : (0,01 - 0,4) $ZrO_2$ : (0,02 - 0,3) $Na_2O$ : (0,03 - 0,6) $R_2O$ : (10 - 40) $H_2O$,

wobei R gleich Tetrapropylammonium ist.

Als Silizium-, Aluminium, Zirkon-, bzw. Natriumverbindungen können beispielsweise eingesetzt werden: Kieselsäuregel, Natriumsilicat, Aluminiumhydroxid, Aluminiumsulfat, Natriumaluminat, Aluminiumhalogenide, Aluminiummetahydroxid, Zirkonhalogenide, Zirkonsulfat, Zirkonylchlorid, Natriumhydroxid, Natriumsulfat, Natriumhalogenide. Aber auch andere Verbindungen der fünf genannten Elemente eignen sich für die Herstellung der Zeolithe.

Das Gemisch der jeweils gewählten Verbindungen mit Wasser wird im allgemeinen 18 bis 360 Stunden, vorzugsweise 24 bis 240 Stunden lang auf eine Temperatur zwischen 100 und 200°C, vorzugsweise zwischen 130 und 170°C, in einem geschlossenen Gefäß erhitzt.

Die gebildeten Zeolithe werden in üblicher Weise, z. B. durch Filtration, isoliert, gewaschen und getrocknet.

Bei dem erfindungsgemäßen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen.

Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen ausgetauschten Formen zeigen katalytische Aktivität.

Die erfindungsgemäßen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete äußere Form zu bringen.

Als Bindermaterialien eignen sich vor allem Oxide oder Hydroxide des Aluminiums und die Oxide bzw. Hydroxide des Siliciums sowie Schichtsilicate, beispielsweise aus der Kaolin- oder Montmorillonit-Familie.

Dieser so hergestellte Zeolith-Katalysator wird gewöhnlich vor dem Einsatz in der erfindungsgemäßen Isomerisierungsreaktion zunächst durch Kalzinieren bei Temperaturen zwischen 300 und 700°C aktiviert. Zur besseren Stabilisierung des Katalysators ist es manchmal vorteilhaft, die Kalzinierung in Gegenwart von Wasserdampf, Ammoniak oder deren Mischungen durchzuführen.

Falls in der Gasphase gearbeitet wird, besteht eine günstige einfache Verfahrensweise zur Durchführung der erfindungsgemäßen Isomerisierung darin, daß man das oder die Toluidine aus einer Dosiervorrichtung zuerst in eine Verdampfungszone und das entstandene Gas dann durch ein von außen beheiztes und mit dem Katalysator gefülltes Reaktionsrohr leitet. Bei Durchführung der Isomerisierung in flüssiger Phase wird das Einsatzmaterial erst erwärmt und dann in flüssiger Form durch das mit dem Katalysator gefüllte Reaktionsrohr geleitet.

Es hat sich im Hinblick auf die Standzeit des Katalysators als vorteilhaft erwiesen, dem Einsatzmaterial zusätzlich noch Wasserstoff oder Wasserdampf zuzumischen.

Darüberhinaus kann es noch vorteilhaft sein, ein unter den Reaktionsbedingungen inertes Trägergas zuzumischen. Als Trägergase eignen sich z. B. Stickstoff und Edelgase.

Wasserstoff, Wasserdampf und/oder das Trägergas werden dabei in einer solchen Menge zugesetzt, daß die Verweilzeit zwischen 1 und 100 s liegt.

Die Vermischung des Wasserstoffs, Wasserdampfs und/oder des Trägergases mit dem Toluidin oder Toluidin-Gemisch erfolgt am vorteilhaftesten in der Verdampfungs- bzw. Erwärmungszone. Es hat sich dabei als vorteilhaft erwiesen, diese Gase vor der Vermischung auf Reaktionstemperatur aufzuheizen.

Die erfindungsgemäße Isomerisierung wird bei Temperaturen zwischen 300 und 550°C, vorzugsweise bei 320 bis 450°C, sowie Drücken von 0,1 bis 30 bar, vorzugsweise bei 2 - 20 bar, insbesondere bei Normaldruck durchgeführt.

Die Belastung des Zeolith-Katalysators - ausgedrückt als LHSV (Liquid Hourly Space Velocity, $h^{-1}$) - liegt im allgemeinen zwischen 0,05 und 10 $h^{-1}$, vorzugsweise zwischen 0,3 und 5 $h^{-1}$.

Die Reaktionsprodukte werden nach Verlassen des Reaktors zur Abtrennung der kondensierbaren Anteile gekühlt. Die erfindungsgemäße Isomerisierung ist jedoch nicht auf diese Verfahrensweise (Festbett-Reaktor) beschränkt, sondern läßt sich im Prinzip auch in anderen geeigneten Reaktor-Typen durchführen (z. B. Wirbelschicht-Reaktor).

Das nicht umgesetzte Ausgangsprodukt kann nach der Trennung durch Destillation, Kristallisation oder Adsorption in den Reaktor zurückgeführt werden.

Sollte die Aktivität des Katalysators mit der Zeit aufgrund einer Verkokung abnehmen, kann er regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650°C über den desaktivierten Katalysator geleitet wird. Stickstoff/Luft wird dabei bevorzugt. Die Temperatur sollte dabei an keiner Stelle des Reaktors 650°C übersteigen. Nach dem Regenerieren besitzt der Katalysator wieder die volle Aktivität.

Toluidine sind wichtige Zwischenprodukte für die Herstellung von Farbstoffen, Vulkanisationsbeschleunigern, Textilhilfsmitteln und zahlreiche weitere Einsatzgebiete.

Die Erfindung soll durch die folgenden Beispiele erläutert werden, wobei die Beispiele aber in keiner Weise einschränkend sein sollen.

## Beispiele

**Beispiel 1** (Isomerisierung von o-Toluidin an zirkonhaltigem Zeolith vom Pentasil-Typ)

a) Katalysator-Herstellung

Zirkonoaluminosilikat vom Pentasil-Typ wurde gemäß Beispiel 1 der EP-A2-0 077 523 wie folgt hergestellt: 16,6 g Natriumaluminat (54 Gew.-% $Al_2O_3$, 41 Gew.-% $Na_2O$) und 14,8 g Natriumhydroxid wurden in 200 g 20 Gew.-%-iger wäßriger Tetrapropylammoniumhydroxid-Lösung gelöst (Lösung A). Eine weitere Lösung (Lösung B) wurde hergestellt, indem man 620 g 40 Gew.-%-iges kolloidales Kieselgel in 2300 g 20 Gew.-%-iger wäßriger Tetrapropylammoniumhydroxid-Lösung löste und diese Lösung am Rotationsverdampfer auf insgesamt 2200 g einengte. Lösung A und Lösung B wurden miteinander vermischt. Zu dieser Mischung wurden unter intensivem Rühren 37,8 g Zirkonylchlorid $ZrOCl_2 \cdot 8H_2O$ gegeben. Die entstandene Suspension wurde homogenisiert und in einem geschlossenen Gefäß 120 h auf 160°C erhitzt. Das entstandene Produkt wurde abfiltriert, mit Wasser gewaschen und bei 120°C getrocknet. Man erhielt 273 g Zirkonoaluminosilicat. Die Röntgenbeugungsanalyse zeigte ein gut kristallines Produkt mit ZSM-5-Struktur.

Anschließend wurde das Pulver 2 Stunden bei 400°C, 3 h bei 450°C und 8 h bei 500°C an Luft calciniert.

In Molverhältnissen der Oxide ausgedrückt, hatte dieses Material die Zusammensetzung:

$SiO_2 : 0{,}035\ ZrO_2 : 0{,}026\ Al_2O_3 : 0{,}023\ Na_2O$.

Es wurde dreimal für einige Stunden bei 100°C mit 1-molarer Ammoniumnitratlösung behandelt, gewaschen, getrocknet und einige Stunden bei 500°C an Luft calciniert.

65 g des so erhaltenen Pulvers wurden mit 35 g $Al_2O_3$ zu Strangpresslingen von 1,6 mm Durchmesser verarbeitet, 4 h bei 500°C calciniert, auf eine Teilchengröße von 0,25 bis 1,0 mm zerkleinert und bei 450°C im Stickstoffstrom zwei Stunden lang calciniert.

b) Isomerisierung

15 ml des gemäß a) hergestellten Katalysators wurden in einen Rohrreaktor aus Glas von 16 mm Innendurchmesser und 50 cm Länge eingefüllt und mit Glaskugeln zum Verdampfen des flüssigen Ausgangsproduktes überschichtet. Die Temperatur in der Katalysatorschüttung konnte mit einem Thermoelement, das sich in der Mitte des Reaktors befand und in axialer Richtung verschiebbar war, gemessen werden. Der Reaktor und die Verdampfungszone befanden sich in einem elektrisch beheizten Ofen. 6 ml/h o-Toluidin wurden über eine Dosierpumpe dem Reaktor zugeführt. 1,5 l/h Wasserstoff wurden über eine Gasversorgung, bestehend aus Reduzierventilen und Vorrichtungen zum Messen des Drucks und der Durchflußmenge, ebenfalls über den Katalysator geleitet. Die kondensierbaren Reaktionsprodukte wurden in einer Kühlfalle bei 0°C kondensiert, ausgewogen und gaschromatographisch analysiert. Tabelle 1 zeigt die Ergebnisse:

**Tabelle 1**: Isomerisierung von o-Toluidin an zirkonhaltigem Pentasil

| Dauer des Versuchs (h) | Temperatur (°C) | Zusammensetzung des Kondensats o-Toluidin (Gew.-%) | m-Toluidin (Gew.-%) | p-Toluidin (Gew.-%) | Anilin (Gew.-%) |
|---|---|---|---|---|---|
| 5 | 340 | 96,7 | 0,1 | <0,1 | 0,1 |
| 8 | 370 | 86,5 | 10,7 | 1,3 | 0,3 |
| 12 | 400 | 62,5 | 28,7 | 8,1 | 0,6 |
| 20 | 400 | 64,7 | 26,5 | 7,3 | 0,6 |
| 31 | 400 | 69,8 | 25,0 | 6,3 | 0,7 |
| 41 | 400 | 70,0 | 24,0 | 6,1 | 0,6 |
| 48 | 400 | 75,1 | 20,5 | 4,6 | 0,5 |
| 49 | 430 | 37,2 | 45,3 | 15,1 | 1,5 |
| 59 | 430 | 44,9 | 39,1 | 11,8 | 1,1 |
| 74 | 430 | 42,1 | 38,5 | 11,4 | 1,0 |
| 104 | 430 | 47,0 | 37,1 | 10,9 | 0,8 |
| 125 | 430 | 48,5 | 36,9 | 11,2 | 0,8 |
| 190 | 430 | 51,2 | 37,0 | 12,0 | 0,7 |

Wie aus der Tabelle ersichtlich ist, desaktivierte der Katalysator bei 400°C zwischen der 12.-ten und der 48.-ten Stunde noch etwas, auch bei 430°C trat zwischen der 49.-ten und der 59.-ten Stunde noch eine geringfügige Desaktivierung auf, aber ab dann war bis zur 190.-ten Stunde kein Nachlassen der Aktivität mehr zu beobachten.

**Vergleichsbeispiel** (Isomerisierung von o-Toluidin an H-ZSM-5)

Diese Isomerisierung wurde unter den gleichen Bedingungen wie in Beispiel 1 durchgeführt, aber mit 15 ml H-ZSM-5 als Katalysator, dessen Herstellung z. B. in US-PS-3 702 886 beschrieben ist. Tabelle 2 zeigt die Ergebnisse:

**Tabelle 2**: Isomerisierung von o-Toluidin an H-ZSM-5

| Dauer des Versuchs (h) | Temperatur (°C) | Zusammensetzung des Kondensats o-Toluidin (Gew.-%) | m-Toluidin (Gew.-%) | p-Toluidin (Gew.-%) | Anilin (Gew.-%) |
|---|---|---|---|---|---|
| 2 | 370 | 86,0 | 11,1 | 2,9 | 0,5 |
| 9 | 400 | 62,0 | 24,3 | 6,5 | 1,1 |
| 23 | 400 | 66,4 | 19,1 | 4,8 | 1,0 |
| 30 | 400 | 71,0 | 18,2 | 4,8 | 1,0 |
| 44 | 400 | 75,2 | 14,3 | 3,5 | 0,7 |
| 51 | 430 | 52,1 | 31,6 | 9,9 | 1,8 |
| 72 | 430 | 67,2 | 22,0 | 8,8 | 1,4 |
| 100 | 430 | 73,9 | 17,7 | 5,8 | 0,9 |
| 128 | 430 | 80,4 | 13,2 | 4,6 | 0,7 |

Der Vergleich mit Beispiel 1 zeigt, daß bereits bei 400°C geringere Ausbeuten an m- und p-Toluidin auftraten, vor allem aber wurde bei 430°C kein konstantes Niveau erreicht, sondern der Katalysator zeigte eine gleichmäßige Desaktivierung zwischen der 51.-ten und 128.-ten Stunde.

**Beispiel 2:**

Dieser Versuch wurde mit dem bereits in Beispiel 1 benutzten Katalysator (ohne vorherige Regenerierung) durchgeführt. 15 ml/h p-Toluidin und 1,5 l/h Wasserstoff wurden bei 430°C über den Katalysator geleitet. Nach einer Vorlaufzeit von 15 Min. zur Einstellung konstanter Betriebsbedingungen wurde für eine Stunde das Kondensat gesammelt. Es bestand aus 14,0 Gew.-% o-, 56,8 Gew.-% m- und 26,4 Gew.-% p-Toluidin sowie 1,2 Gew.-% Anilin.

**Patentansprüche**

1. Verfahren zur Isomerisierung von o-, m- oder p-Toluidin oder einem Gemisch derselben an einem Zeolith-Katalysator, dadurch gekennzeichnet, daß ein zirkonhaltiger Zeolith vom Pentasil-Typ bei Temperaturen zwischen 300 und 550°C und Drücken zwischen 0,1 bar und 30 bar verwendet wird.

2. Verfahren zur Herstellung von m-Toluidin durch Isomersierung von o- oder p-Toluidin oder einem Gemisch derselben an einem Zeolith-Katalysator, dadurch gekennzeichnet, daß ein zirkonhaltiger Zeolith vom Pentasil-Typ bei Temperaturen zwischen 300 und 550°C und Drücken zwischen 0,1 bar und 30 bar verwendet wird.

3. Verfahren nach Anspruch 1 oder 2, dadurch gekennzeichnet, daß der Zeolith Protonen als Kationen enthält.

4. Verfahren nach einem der Ansprüche 1 bis 3, dadurch gekennzeichnet, daß die Isomerisierung in Gegenwart von Wasserstoff, Stickstoff, Wasserdampf, Argon oder einem Gemisch aus diesen durchgeführt wird.

5. Verfahren nach einem der Ansprüche 1 bis 3, dadurch gekennzeichnet, daß die Isomerisierung in Gegenwart von Wasserstoff durchgeführt wird.

**Claims**

1. A process for the isomerization of o-, m- or p-toluidine, or of a mixture of them, on a zeolite catalyst, which comprises use of a zirconium-containing zeolite of the pentasil type at temperatures between 300 and 550°C and pressures between 0.1 bar and 30 bar.

2. A process for the preparation of m-toluidine by isomerization of o- or p-toluidine, or of a mixture of them, on a zeolite catalyst, which comprises use of a zirconium-containing zeolite of the pentasil type at temperatures between 300 and 550°C and pressures between 0.1 bar and 30 bar.

3. The process as claimed in claim 1 or 2, wherein the cations contained in the zeolite are protons.

4. The process as claimed in one of claims 1 to 3, wherein the isomerization is carried out in the presence of hydrogen, nitrogen, steam, argon or of a mixture of these.

5. The process as claimed in one of claims 1 to 3, wherein the isomerization is carried out in the presence of hydrogen.

**Revendications**

1. Procédé pour isomériser l'o-toluidine, la m-toluidine ou la p-toluidine ou un mélange de celles-ci en présence d'un catalyseur qui est une zéolite, procédé caractérisé en ce qu'on utilise une zéolite contenant du zirconium du type des pentasils, à des températures comprises entre 300 et 550°C et sous des pressions comprises entre 0,1 bar et 30 bar.

2. Procédé pour préparer la m-toluidine par isomérisation de l'o-toluidine ou de la p-toluidine ou d'un mélange de celles-ci, en présence d'un catalyseur qui est une zéolite, procédé caractérisé en ce qu'on utilise une zéolite contenant du zirconium du type des pentasils, à des températures comprises entre 300 et 550°C et sous des pressions comprises entre 0,1 bar et 30 bar.

3. Procédé selon l'une des revendications 1 et 2, caractérisé en ce que la zéolite contient des protons comme cations.

4. Procédé selon l'une quelconque des revendications 1 à 3, caractérisé en ce que l'isomérisation est effectuée en présence d'hydrogène, d'azote, de vapeur d'eau, d'argon ou d'un mélange de ceux-ci.

5. Procédé selon l'une quelconque des revendications 1 à 3, caractérisé en ce que l'isomérisation est effectuée en présence d'hydrogène.